# EUROPEAN PATENT APPLICATION

(11) **EP 2 674 173 A1**
(43) Date of publication of application: **18.12.2013**
(21) Application number: 12172281.3
(22) Date of filing: 15.06.2012
(51) Int. Cl.: A61L 27/34, A61F 9/008, A61L 27/54

(54) **Implantable material grafted with a cell antiproliferative and/or antibacterial film and process of grafting thereof**

(71) Applicant: COMMISSARIAT A L'ENERGIE ATOMIQUE ET AUX ENERGIES ALTERNATIVES, 75015 Paris (FR); Biowintech, 75006 Paris (FR)
(72) Inventor: Deniau, Guy, 78690 Les Essarts-le-roi (FR); Oudin, Maxime, 75002 Paris (FR); Brouzes, Alexandre, 91190 Gif Sur Yvette (FR); David, Laurent, 75013 Paris (FR)
(74) Representative: Icosa

(57) **Abstract**

The present invention relates to an implantable material having at least one external surface grafted with a polymeric film comprising carboxylate and sulfonate functions wherein the film is produced by copolymerization-like reaction of a source of carboxylate functions and a source of sulfonate functions. The invention also relates to a process for grafting of a polymeric film according to the invention onto at least one external surface of an implantable material. The invention further relates to the use of a grafted implantable material for the manufacture of an antiproliferative and/or antibacterial implantable medical device.

## Description

### FIELD OF INVENTION

The present invention relates to an implantable material whose surface is grafted with a cell antiproliferative and/or antibacterial film. Especially, the present invention relates to an intraocular lens (IOL) whose surface is grafted with a cell antiproliferative and/or antibacterial film. The cell antiproliferative and/or antibacterial film of the invention comprises free carboxylate and sulfonate functions. The present invention also relates to a process for grafting a cell antiproliferative and/or antibacterial film at the surface of an implantable material, preferably an IOL.

### BACKGROUND OF INVENTION

Most of materials that are implanted in the organism or simply transiting inside thereof need to be at least biocompatible. Implantable materials often need to further have antirejection, cell antiproliferation and antibacterial properties.

For example, in order to avoid restenosis after stent implantation in an artery, the stent may have antirejection and/or antiproliferation properties. Chemists have developed a coating that may be physisorbed on the stent, said coating comprising an antirejection drug that is sustainingly released in blood.

Another example pertains to the field of ocular implants for which cell antiproliferation and antibacterial properties may be needed. Indeed, in order to avoid cataract, the sole efficient treatment consists in a surgical operation during which the crystalline lens is replaced by an intraocular implant (IOL). The most frequent post-operative complication of this treatment is the development of a cicatricial tissue around the implant, leading to an opacification and that is called secondary cataract. A second surgical operation is thus needed to solve this opacification problem. This side effect appears in 38% of patients about 9 years after the implantation of the IOL. Solutions are therefore expected to avoid secondary cataract and are searched through three main axes:
- improvement of surgical methods: more precise methods are tested, especially in order to reduce the size of the incision and therefore avoid ocular traumatisms;
- study of the implant geometry : it has been shown that certain forms of lens provide a mechanical barrier that avoid cell migration between the implant and the eye capsule;
- use of new materials and/or surface treatments in order to improve biocompatibility and/or to bring new functionalities.

Intraocular lens are mainly obtained from 3 types of materials: silicones, hydrophobic methacrylates and hydrophilic methacrylates. These materials are biocompatible and have convenient physical and optical properties. However, these materials do not have particular cell antiproliferation or antibacterial properties. Therefore, the development of new material or surface treatment bringing cell antiproliferation and/or antibacterial properties to IOL is needed.

More generally, it is a general concern that implantable materials shall have cell antiproliferation and/or antibacterial properties in order to avoid colonization or infection when implanted..

It was described in the prior art that certain chemical functions, when present at the surface of materials, may give cell antiproliferation and/or antibacterial properties to said material. Especially, mimicking heparin by using the association of carboxylate and sulfonate functions, in specific ratios, may confer cell antiproliferation and/or antibacterial properties to the substrate on which it is present. Coatings comprising carboxylate and sulfonate functions are for example described in US patent US6,248,811. Depending on the value of the molar ratio of carboxylate functions to sulfonate functions, the coating polymers may have antibacterial properties, an antibacterial action together with cell proliferation-inhibiting properties or cell proliferation-promoting properties. In patent US6,248,811, the polymer is first synthesized and isolated and is then grafted under UV radiative induction on the surface to be coated.

US6,218,492 discloses a polymer, which is water-insoluble, and is bacteriophobic or inhibits cell proliferation. This polymer is produced by free-radical copolymerization of component I (containing a carboxyl group), component II (containing a sulfonic acid group), and component III (which is an aliphatically unsaturated monomer, but is not acrylonitrile and vinylidene chloride) and wherein from 0.5 to 30 mol % of the polymer is derived from component I and component II. This polymer has antibacterial and/or antiproliferative properties and may either be used as implantable constitutive material or being coated at the surface of an implant. However, this polymer presents low mechanical properties that render it difficult to be processed as constitutive material and affect its resistance overtime when coated as a layer.

Yammine et al. also described coatings comprising carboxylate and sulfonate functions (Yammine et al., Biomacromolecules, 2005, 6(5), 2630-2637). Especially, they developed photo-crosslinkable polymers bearing cinnamic, sulfonate and carboxylate functions to coat silicon intraocular lenses in order to reduce "secondary cataract" by inhibiting cell proliferation. The polymer is first synthesized by radical polymerization and then grafted on the IOL by cycloaddition reaction of photosensible groups.

Methods presented above present the inconvenient to be at least two-steps methods, requiring first the synthesis of the polymer and then its grafting on the surface of the material.

Coury et al. described heparin-like material and surfaces made by co-polymerization of acrylic acid (AA) and 2-acrylamido-2-methyl propane sulfonic acid (AMPS) in order to decrease bacterial and platelet adherence in patent US5,278,200. The polymer may be first synthesized and then grafted on the material. The polymer may also be directly grafted on the material by the generation of free radicals on the material surface (such as polyurethane surface), using Ce(IV) ions and radical copolymerization of AA and AMPS. However, grafting using Ce(IV) ions at the surface of the material presents, among others, the following drawbacks:
- the method is not versatile as it may only be performed on materials comprising oxidizable functions at their surfaces;
- cerium ions remain at the surface of the grafted material, which is incompatible with biological applications as cerium ions are toxic;
- the polymerization should be performed under controlled atmosphere, need an energy intake as it has to be performed at 40°C and is slow, usually at least 3 hours, in other words this method is not easily industrializable.

Therefore, there is a need to develop simple and reproducible methods to graft appropriate ratios of carboxylate and sulfonate functions at the surface of implantable materials, especially organic implantable material, more preferably IOLs, to give cell antiproliferation and/or antibacterial properties. Carboxylate and sulfonate function need to be chemically grafted on the substrate, and not simply adsorbed, in order to ensure a greater functional longevity to the material.

However, to the knowledge of the Applicant, no simple and reproducible method exists to chemically graft carboxylate and sulfonate functions in controlled ratios and controlled thickness on materials used for implantation within a living body, especially on organic implantable materials, more specifically on materials used for IOLs.

The present invention provides a simple and efficient process to chemically graft carboxylate and sulfonate functions on implantable materials, preferably on IOLs.

There are currently a number of techniques available making it possible to chemically graft a coating on a substrate each based on a suitable family or class of molecules as well as specific substrates in some cases.

Techniques for forming an organic coating grafted at the surface of a support, such as photochemical initiation or plasma deposition, as described for example in the articles of Konuma M. ("Film deposition by plasma techniques", 1992, Springer Verlag, Berlin) and Biederman H. and Osada Y. ("Plasma polymerization processes", 1992, Elsevier, Amsterdam), are based on the same principle: generating, near the surface to be covered, unstable forms of a precursor, which evolve by forming a film on the substrate. While the plasma deposition requires no particular properties of its precursors, the photo-initiation necessitates the use of photosensitive precursors, the structure of which evolves under light irradiation. These techniques generally give way to the formation of adherent films, although it is usually impossible to discern whether this adhesion is due to a cross-linking of a film topologically closed around the object or to a real formation of bonds at the interface. These methods present the inconvenient to require relatively complex and costly pre-treatments, the use of vacuum set-ups for the plasma methods or the use of irradiation devices for photochemical initiation.

The self-assembly of monolayers is a very simple technique to implement (Ulman A., "An introduction to ultrathin organic films from Langmuir-Blodgett films to self-assembly", 1991, Boston, Academic Press). However, this technique requires the use of molecular precursors having an adequate affinity for the surface to be grafted. This technique requires determining a precursor-surface pair, such as sulphur compounds having an affinity for gold or silver, tri-halogenosilanes for oxides such as silica or alumina, and polyaromatics for graphite or carbon nanotubes. In each case, the formation of the film is based on a specific chemical reaction between a portion of the molecular precursor and some "receptor" sites on the surface. A chemisorption reaction ensures the adhesion. At room temperature and in solution, films of molecular thickness (less than 10 nm) are obtained. However, while the pairs involving oxide surfaces give way to the formation of very solidly grafted films, this is not the case for surfaces without oxide. In these cases, the interface bond is fragile and the monolayer may desorb when heated or contacted with a suitable solvent at room temperature, or when they are placed in contact with an oxidizing or reducing liquid medium.

The electrografting of polymers is a technique based on the electrically induced initiation, followed by polymerization by chain propagation, of electroactive monomers on the surface of interest acting both as electrode and polymerization primer (Palacin et al., Chemphyschem, 2004, (5)10, 1469-1481). The electrografting requires the use of precursors suitable for its mechanism of initiation by reduction and propagation, generally anionic because cathodically-initiated electrografting is often preferred, applicable on noble and non-noble metals (unlike the electrografting by anodic polarization, which is applicable only on noble or carbon substrates: graphite, vitreous carbon, boron-doped diamond). The "depleted vinyl" molecules, i.e. bearing electroattractive functional groupings, such as acrylonitriles, acrylates and vinyl-pyridines, are particularly suitable for this method, which allows for numerous applications in the microelectronics or biomedical field. The adherence of these electrografted films is ensured by a carbon-metal covalent bond (Deniau et al., Surf. Sci., 2006, 600, 675-684).

Among the various techniques mentioned above, electrografting is the only technique that makes it possible to produce grafted films with a specific control of the bonding interface. Indeed, the only technique making it possible to graft polymer films resulting from vinyl monomers activated on surfaces, which are necessarily conductive, consists of electrically initiating the polymerization reaction from the surface via a potentiostat, followed by a growth of chains, monomer-by-monomer. This method presents the drawback to require the use of an electrochemical cell with a cathode and an anode, as well as an application of a voltage at the terminals thereof.

Relative to the electrografting technic, Ortiz et al. (Ortiz et al., Journal of Electroanalytical Chemistry, 1998, 455, 75-81) described the grafting of diazonium salts synthesized in situ in the aqueous acid phase by electrochemical initiation. International patent application WO 03 018212 describes, in particular, a method for grafting and growing an organic conductive film on an electrically conductive surface, the grafting and the growth being performed simultaneously by electroreduction of a diazonium salt that is a precursor of said organic film.

A method was recently developed that differs from above methods in that it makes it possible to perform the grafting of organic polymer or copolymer films in the absence of an electric voltage (EP 2 121 814). This method, used under the name Graftfast^{®}, makes it possible to graft films onto surfaces of various types, and its application is not limited to electrically conductive or semi-conductive surfaces.

The Graftfast^{®} method enables chemically grafting an organic film at the surface of a solid support. The method is based on chemical reactions, essentially copolymerization-like reaction.

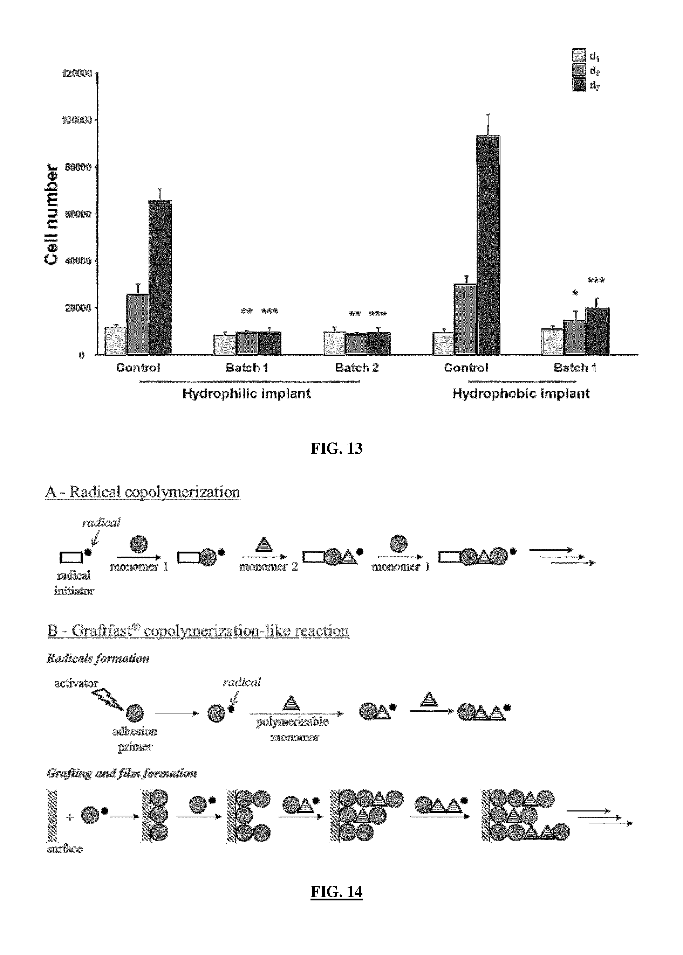

In radical copolymerization, a first monomer is added on a radical initiator to form a radical building block. Further monomers, identical or different, are then successively added on the growing free radical copolymer as represented on figure 14-A.

Contrary to radical copolymerization, in which the growing copolymer bears a radical that react with the non-radical monomer, the copolymerization-like reaction of Graftfast^{®} require the use of an activator to generate a radical entity at each step the growing of the copolymer (figure 14-B). The adhesion primer, activated under the form of a radical, first reacts with the grafted surface. Further adhesion primers activated under the form of radicals then react with grafted adhesion primer. Polymerizable monomers may also be used in order to obtain a copolymer. In this case, an adhesion primer activated under the form of a radical reacts with the polymerizable monomer to form a radical building bloc that can then be added on the grafted surface.

Therefore, the Graftfast^{®} method is implemented on a substrate using an adhesion primer, in a solvent, in presence of an activator and optionally in presence of polymerizable monomers.

The adhesion primer corresponds to one or several monomer species which will form an organic polymer film anchored at the surface of the substrate. Generally, the adhesion primer includes diazonium salts which strong reactivity ensures a robust covalent link between the polymer film and the substrate. The reaction of the diazonium salts with a chemical activator having reducing properties allows the reduction of the diazonium and generation of radicals. The activator may therefore be a chemical agent but it may also be a physical condition, such as for example a given temperature or a photoactivation.

The Graftfast^{®} method had never been used to graft carboxylate and sulfonate functions in controlled ratio on implantable materials.

One limiting aspect of the Graftfast® technology is the difficulty to determine conditions leading to a reproducible film.

The Applicant performed an extensive research work to make it possible to carry out the Graftfast® technology for this specific goal of providing carboxylate and sulfonate grafted surfaces in a reproducible and controlled manner. This invention thus relates to an implantable material grafted at the surface thereof with a polymeric film comprising carboxylate and sulfonate functions wherein the film is produced by copolymerization-like reaction of a source of carboxylate functions and a source of sulfonate functions.

As a further result of this research, the Applicant shows that, surprisingly, the use of an aromatic adhesion primer together with an aromatic polymerizable monomer gives satisfactory results.

Therefore, the present invention relates to an implantable material grafted with a cell antiproliferative and/or antibacterial film comprising carboxylate and sulfonate functions obtained by copolymerization-like reaction by contacting the surface of the material with a composition comprising a source of carboxylate functions and a source of sulfonate functions.

### SUMMARY

The present invention relates to an implantable material having at least one external surface grafted with a polymeric film comprising carboxylate and sulfonate functions wherein the film is produced by copolymerization-like reaction of a source of carboxylate functions and a source of sulfonate functions.

According to one embodiment, one source of carboxylate functions is an aromatic adhesion primer comprising at least one carboxylic acid function or at least one carboxylate salt function.

According to one embodiment, one source of sulfonate functions is an aromatic polymerizable monomer, preferably an aromatic polymerizable vinylic monomer comprising at least one sulfonic acid function or at least one sulfonate salt function.

According to one embodiment, one source of carboxylate functions is a diazonium salt used as adhesion primer, preferably a carboxybenzene diazonium salt, preferably a 4-carboxybenzene diazonium salt, more preferably sodium 4-carboxybenzene diazonium or 4-carboxybenzene diazonium tetrafluoroborate.

According to one embodiment, one source of sulfonate functions is a styrene derivative use as polymerizable vinylic monomer, preferably a styrene sulfonate salt, preferably sodium 4-styrene sulfonate.

According to one embodiment, the film is produced by copolymerization-like reaction of a 4-carboxybenzene diazonium salt and a styrene sulfonate salt, preferably by copolymerization-like reaction of sodium 4-carboxybenzene diazonium and sodium 4-styrene sulfonate or of 4-carboxybenzene diazonium tetrafluoroborate and sodium 4-styrene sulfonate.

According to one embodiment, the thickness of the polymeric film is ranging from 1 nm to 50 nm, preferably from 2 nm to 20 nm.

According to one embodiment, the ratio between the number of carboxylate functions and sulfonate functions [COO⁻]/[SO₃⁻] is less than 10, preferably less than 5, more preferably ranging from 0.5 to 2, more preferably ranging from 0.7 to 1.3, more preferably ranging from 0.9 to 1.1.

The present invention also relates to a process for grafting of a polymeric film onto at least one external surface of an implantable material, comprising a step of contacting said external surface with a solution comprising a source of carboxylate functions, preferably a carboxybenzene diazonium salt, and a source of sulfonate functions, preferably a styrene sulfonate salt, in a solvent, under conditions enabling the formation of radical entities.

According to one embodiment, the solvent is water, deionized water, distilled water, acidified or not, acetic acids, hydroxylated solvents such as ethanol, low-molecular-weight liquid glycols such as ethyleneglycol and mixtures thereof.

According to one embodiment, the conditions enabling the formation of radical entities comprise the use of a reducing agent, preferably ascorbic acid.

According to one embodiment, the carboxybenzene diazonium salt is obtained in situ from aminobenzoic acid by a diazotation reaction, preferably in presence of sodium nitrite.

The invention further relates to the use of an implantable material according to the invention for the manufacture of an antiproliferative and/or antibacterial implantable medical device, preferably an antiproliferative and/or antibacterial implant, more preferably an antiproliferative and/or antibacterial intraocular lens.

The invention also relates to a kit comprising an implantable material according to the invention and an inserting and/or implantation device, preferably wherein the implantable material is an intraocular lens and the implantation device is an IOL-inserting system.

### DEFINITIONS

In the present invention, the following terms have the following meanings:
- **"implantable material"** refers in the meaning of the present invention to a material and/or a medical device that is at least biocompatible and may be introduced and left within a living body without triggering immune reactions.
- **"implantable medical device"** refers to a medical device that is at least biocompatible and may be introduced and left within a living body without triggering immune reactions and may be for example implants, intraocular lenses (IOLs), stents, catheters, implants for abdominal surgery, vascular prostheses, artificial limbs.

- **"implantation device"** or **"inserting device"** refers to a device that is used to insert an implantable medical device within a living body. Especially, an intraocular lens may be implanted using IOL-inserting device.
- **"grafted surface"** refers to a surface on which a coating is chemically anchored. In the present invention, a grafted surface should be understood in contrast with a coated surface, wherein the coating is just adsorbed onto.
- **"organic film"** or **"polymeric film"** refers to any polymer or copolymer film, resulting from the polymerization of a plurality of monomer units of identical or different chemical species and adhesion primer molecules. The films obtained by the method of the present invention are essentially of the copolymer type insofar as the film incorporates species resulting from the adhesion primer and from polymerizable monomers.
- **"copolymerization-like reaction"** refers to a method of by which a polymer is formed by the successive addition of free radical building blocks. In the present invention, the copolymerization-like reaction is performed in presence of an adhesion primer and of an activator. In one embodiment, the copolymerization-like reaction is performed in presence of at least two different adhesion primers and of an activator. In another embodiment, the copolymerization-like reaction is performed in presence of at least one adhesion primer, of at least one polymerizable monomer and of an activator.
- **"adhesion primer"** refers to an organic molecule capable, under certain conditions, of being chemisorbed at the surface of a solid substrate by a radical chemical grafting, and comprising a reactive function with respect to another radical after chemisorption.
- **"polymerizable monomer"** refers to an organic molecule comprising a functional moiety, capable, under certain conditions, to be used as a monomer for the synthesis of a polymer. In a preferred embodiment of the present invention, the polymerizable monomer is a polymerizable vinylic monomer.
- **"polymerizable vinylic monomer"** refers to an organic molecule comprising a vinyl moiety, capable, under certain conditions, to be used as a monomer for the synthesis of a polymer.
- **"activator"** refers to a chemical compound, such as a compound with reducing properties, or a physical condition, such as temperature or photoactivation, that allows the initiation of copolymerization-like reaction.
- **"conditions enabling the formation of radical entities"** comprise the use of an activator according to the present invention.
- **"protic solvent"** refers to a solvent that comprises at least one hydrogen atom capable of being released in proton form.
- **"source of carboxylate functions"** refers to a chemical compound comprising at least one carboxylic acid function or at least one carboxylate salt function.
- **"source of sulfonate functions"** refers to a chemical compound comprising at least one sulfonic acid function or at least one sulfonate salt function.
- **"carboxylate function"** refers to the chemical formula -COO⁻.
- **"carboxylic acid function"** refers to the chemical formula -COOH.
- **"carboxylate salt"** refers to the formula -COO⁻X⁺ wherein X is an inorganic or organic cation, preferably sodium, potassium, magnesium, calcium.
- **"sulfonate function"** refers to the chemical formula -SO₃⁻.
- **"sulfonic acid function"** refers to the chemical formula -SO₃H.
- **"sulfonate salt"** refers to the formula - SO₃⁻X⁺ wherein X is an inorganic or organic cation preferably sodium, potassium, magnesium, calcium.
- **"diazonium salt"** refers to an organic compound comprising a -N₂⁺X⁻ functional group wherein X is an inorganic or organic anion, preferably Cl⁻ or BF₄⁻.
- **"cell antiproliferative"** or **"that inhibit cell proliferation"** refers to the property of at least limiting cell colonization, i.e. limiting adhesion and/or multiplication of cells, on the surface that has cell antiproliferative properties. In the present invention, a substrate whose surface is grafted or coated with a cell antiproliferative film refers to the fact that said film has the property to limit cell colonization on said surface.
- **"antibacterial"** refers to the property of limiting bacteria proliferation. In the present invention, a substrate whose surface is grafted or coated an antibacterial film refers to the fact that said film has the property to limit bacteria proliferation on said surface.
- **"cytostatic"** refers to the property to prevent cell growth.
- **"cytotoxic"** refers to the property to induce cell death.
- **"about"** preceding a figure means plus or less 10% of the value of said figure.

In the present invention and unless otherwise stated, the normal conditions of temperature and pressure correspond to a temperature of 25°C and to a pressure of 1.10⁵Pa.

### DETAILED DESCRIPTION

### Grafted implantable material

The present invention relates to an implantable material grafted with a polymeric film comprising carboxylate and sulfonate functions wherein the film is produced by copolymerization-like reaction of at least one source of carboxylate functions and at least one source of sulfonate functions.

In one embodiment, the polymeric film of the invention is produced by copolymerization-like reaction of at least one source of carboxylate functions and at least one source of sulfonate functions, one of which being an adhesion primer and the other being a polymerizable monomer. In a preferred embodiment, the polymeric film of the invention is produced by copolymerization-like reaction of at least one source of carboxylate functions and at least one source of sulfonate functions, one of which being an adhesion primer and the other being a polymerizable monomer, said copolymerization-like reaction being performed in presence of an activator.

In one embodiment, the polymeric film of the invention is produced by copolymerization-like reaction of at least one adhesion primer which is a source of carboxylate functions, preferably a 4-carboxybenzene diazonium salt, and at least one polymerizable vinylic monomer which is source of sulfonate functions, preferably a styrene sulfonate salt. In a preferred embodiment, the polymeric film of the invention is produced by copolymerization-like reaction of at least one adhesion primer which is a source of carboxylate functions, preferably a 4-carboxybenzene diazonium salt, and at least one polymerizable vinylic monomer which is source of sulfonate functions, preferably a styrene sulfonate salt, said copolymerization-like reaction being performed in presence of an activator.

According to one embodiment, the 4-carboxybenzene diazonium salt is 4-carboxybenzene diazonium tetrafluoroborate or 4-carboxybenzene diazonium chloride.

According to one embodiment, the styrene sulfonate salt is sodium 4-styrene sulfonate.

According to one embodiment, the implantable material that is grafted in the present invention is an implantable medical device, preferably an intraocular lens (IOL).

According to another embodiment, the implantable material that is grafted in the present invention comprise at least one surface comprising silicone, polysiloxane, perfluoroalkyl polyether, acrylates such as polymetacrylates, polyacrylates, fluorinated polymethacrylate or polyalkylmethacrylate, polyamides, fluorinated polyolefin, polyhydroxyethylmethacrylate (PHEMA), polyethylene (PE), polypropylene (PP), polyethylene tetraphtalate (PET), polytetrafluoroethylene (PTFE), polyurethanes.

In an embodiment, the grafted film obtained in the present invention is a copolymer. According to another embodiment, the grafted film obtained in the present invention is a ter-polymer.

According to one embodiment, the grafted implantable material of the invention is cytostatic. In other words, the polymeric film grafted at the surface of the implantable material of the present invention is a cell antiproliferative film.

According to one embodiment, the cell proliferation is reduced from a percentage ranging from 50% to 100 %, preferably from 80 to 100%, more preferably from 90 to 100% on the grafted surface of the implantable material of the invention compared to a non-grafted surface of the same material.

Cell proliferation may be measured by cell counting using MTT (3-(4,5-dimethylthiazol-2-yl)-2,5-diphenyltetrazolium bromide) colorimetric assay.

According to a further embodiment, the grafted implantable material of the invention is not cytotoxic.

According to one embodiment, the grafted implantable material of the invention has antibacterial properties.

According to one embodiment, the number of bacteria is reduced from a percentage ranging from 80% to 100 %, preferably from 90 to 100% on the grafted surface of the implantable material of the invention compared to a non-grafted surface of the same material.

According to one embodiment, the grafted implantable material of the invention has cell antiproliferative and antibacterial bacterial properties.

According to a specific embodiment, the grafted polymeric film of the invention prevents the proliferation of cells and/or of bacteria on the surface of the grafted implantable material, especially it prevents the proliferation of lens cells, endothelial cells, keratinocytes or fibroblasts.

According to one embodiment, the grafted polymeric film of the invention presents a ratio of carboxylate functions over sulfonate functions [-COO⁻]/[-SO₃⁻] of less than 10, preferably less than 5, more preferably ranging from 0.5 to 2, more preferably ranging from 0.7 to 1.3, more preferably ranging from 0.9 to 1.1.

In one embodiment, the ratio [-COO⁻]/[-SO₃⁻] of the film of the invention is ranging from 0 to 7, preferably from 0 to 5 and the polymeric film has pronounced antibacterial properties.

In one embodiment, the ratio [-COO⁻]/[-SO₃⁻] of the film of the invention is ranging from 0 to 5, preferably from 0 to 3 and the polymeric film has pronounced cell antiproliferative properties.

In one embodiment, the ratio [-COO⁻]/[-SO₃⁻] of the film of the invention is ranging from 0,2 to 2, preferably from 0,5 to 1,5 and the polymeric film has pronounced antibacterial properties together with cell antiproliferative properties.

According to one embodiment, the ratio [-COO⁻]/[-SO₃⁻] of the film of the invention may be controlled by varying the concentration in polymerizable monomer and/or adhesion primer used for the copolymerization-like reaction.

According to one embodiment, the ratio [-COO⁻]/[-SO₃⁻] of the film of the invention may be determined by measuring the zeta potential of the grafted surface of the material, preferably using an Anton Paar SurPASS zetameter.

According to one embodiment, the grafted polymeric film of the present invention has a mechanical resistance to friction strength up to 15 bars.

According to one embodiment, the grafted polymeric film of the present invention has a mechanical resistance to folding.

In the case wherein the implantable material of the invention is an IOL, mechanical resistance to friction and/or folding of the grafted film may be determined by passing the grafted IOL through an insertion cartridge usually used to inject IOLs in the eye and then analyzing the grafted film.

According to an embodiment, the thickness of the grafted polymeric film of the invention is ranging from 1 nm to 50 nm, preferably from 2 nm to 20 nm. According to one embodiment, the thickness of the polymeric film may be measured by IR spectrometry, using an IR Abacus.

According to one embodiment, the surface of the implantable material is totally covered by the polymeric film. According to another embodiment, the surface of the implantable material is partially covered by the polymeric film. According to one embodiment, the percentage of the surface of the implantable material that is covered by the polymeric film is ranging from 40 to 100, preferably from 70 to 100 and may be determined by weighting, by punctual measures in different points of the surface, or by XPS analysis.

According to one embodiment, the polymeric film is uniform, i.e. it has a homogenous surface over the entire surface of the grafted implantable material.

### Process

The present invention also relates to the process for grafting a cell antiproliferative and/or antibacterial polymeric film at the surface of an implantable material, preferably an IOL.

According to one embodiment, the process of the invention is a process for grafting a polymeric film comprising carboxylate and sulfonate functions onto the surface of an implantable material, preferably at the surface of an implantable medical device, preferably at the surface of an IOL.

According to one embodiment, the process of the present invention comprises a step of contacting the surface of the implantable material with a solution comprising at least one source of carboxylate functions and at least one source of sulfonate functions in a solvent, under conditions enabling the formation of radical entities.

According to one embodiment, the solution used in the process of the invention comprises at least one source of carboxylate functions and at least one source of sulfonate functions, wherein one of which is an adhesion primer and the other is a polymerizable monomer.

According to one embodiment, one source of carboxylate functions is an adhesion primer, preferably a 4-carboxybenzene diazonium salt. According to one embodiment, one source of sulfonate functions is a polymerizable monomer, preferably a polymerizable vinylic monomer, more preferably a styrene sulfonate salt.

According to one embodiment, one source of carboxylate functions is a 4-carboxybenzene diazonium salt and one source of sulfonate functions is a styrene sulfonate salt.

According to one embodiment, the source of carboxylate functions is free of acrylic acid.

Without willing to be linked by any theory, the Applicant proposes that the mechanism of the grafting reaction first comprises the formation of radicals from the adhesion primer. The radicals' formation may be initiated in presence of a chemical activator and/or physical conditions. According to one embodiment, the radicals' formation is initiated by a chemical activator, for example by a reducing agent.

In the case wherein the adhesion primer is a diazonium salt, when the diazonium salt is reduced to form a radical, there is at the same time a nitrogen release. According to another embodiment, the radicals formation is initiated by a physical conditions, for example by using a specific temperature or by illumination at a given wave length. It is then supposed that the radicals get grafted on the surface of the material to form a primary layer of adhesion. At the same time, radicals formed from the adhesion primer initiate the radical polymerization of the polymerizable monomer. Growing polymeric chains of polymerizable polymer then graft themselves to the surface of the material, on the primary layer, to form the copolymer film (see figure 14-B).

According to one embodiment, materials that may be grafted by the process of the present invention may have a surface comprising silicone, polysiloxane, perfluoroalkyl polyether, acrylates such as polymetacrylates, polyacrylates, fluorinated polymethacrylate or polyalkylmethacrylate, polyamides, fluorinated polyolefin, polyhydroxyethylmethacrylate (PHEMA), polyethylene (PE), polypropylene (PP), polyethylene tetraphtalate (PET), polytetrafluoroethylene (PTFE), polyurethanes.

According to one embodiment, material that may be grafted by the process of the present invention may be under the form of a constitutive block, of a woven or nonwoven textile, it may be full or empty.

According to one embodiment, the amount of adhesion primer, in the solution used in the process of the present invention may vary as desired by the experimenter. Variations of this amount may participate to the control of the thickness of the grafted film. From the amount of the adhesion primer in the solution may also depends the amount of adhesion primer integrated in the organic film and therefore it may influence the [-COO⁻]/[-SO₃⁻] ratio. In order to obtain a film grafted on the quite entire surface of the material, it is necessary to use a minimum amount of adhesion primer which may be estimated by molecular size calculation together with the size of the surface to be grafted.

According to one embodiment, the concentration of adhesion primer in the solution used in the process of the present invention is ranging from 0.005 M to 0.2 M, preferably from 0.01 M to 0.1 M, more preferably from 0.02 to 0.08 M, more preferably about 0.05M.

According to one embodiment, the solution of adhesion primer is an acidic solution. In this embodiment, the pH of the solution is ranging from 1 to 7, preferably from 2 to 5.

The adhesion primer may either be directly introduced in the solution used in the process of the present invention or be prepared in situ in the latter. When the adhesion primer is prepared in situ, the reaction is referred to as a "one-pot" reaction.

In one embodiment, the adhesion primer is a diazonium salt, preferably a 4-carboxybenzene diazonium salt. According to a first embodiment, the process of the invention comprises a step of preparing the 4-carboxybezene diazonium salt by reacting the 4-aminobenzoic acid with NaNO₂ in an acidic medium. For detailed experimental method that may be used for such an *in situ* preparation, one skilled artisan can refer to Lyskawa and Belanger, Chem. Mater. 18, 2006, 4755-4763. The grafting will then preferably be performed directly in the solution used for the preparation of the diazonium salt.

According to a second embodiment, the diazonium salt is directly introduced in the solution used in the process of the present invention. In one embodiment, a 4-carboxybezene diazonium salt may have been separately obtained by reacting a 4-aminobenzeoic acid with boron trifluoride diethyl etherate in presence of tert-butyl nitrite and isolating the resulting 4-carboxybezene diazonium tetrafluoroborate. The skilled artisan may also refers to other known methods to synthesize and isolate diazonium salts in order to obtain 4-carboxybezene diazonium salt.

According to one embodiment, the adhesion primer is preliminary dissolved in the solvent of the reaction prior use.

According to an embodiment, the radically polymerizable monomer implemented in the process of the present invention is a vinylic monomer, preferably a styrene sulfonate salt, more preferably sodium styrene sulfonate. According to a specific embodiment, the monomer used in the process of the invention is a mixture comprising sodium styrene sulfonate and at least one other radically polymerizable monomer, such as for example styrene, acrylate or metacrylate. The invention also applies to a mixture of two, three, four or more monomers comprising sodium styrene sulfonate and another monomer selected from styrene, acrylate or metacrylate.

The amount of polymerizable monomer in the solution used in the process of the present invention may vary as desired by the experimenter. Variations of this amount may participate to the control of the thickness of the grafted film. The amount of the polymerizable monomer may also influence the [-COO⁻]/[-SO₃⁻] ratio.

According to one embodiment, the concentration of polymerizable monomer in the solution used in the process of the present invention is ranging from 0.05 M to 5 M, preferably from 0.1 M to 2 M, more preferably from 0.2 M to 1 M.

In one embodiment, the ratio of carboxylate functions over sulfonate functions [-COO⁻]/[-SO₃⁻] may be controlled by varying the concentration in polymerizable monomer and/or adhesion primer. According to one embodiment, the ratio of carboxylate functions over sulfonate functions [-COO⁻]/[-SO₃⁻] is controlled by varying the concentration in styrene sulfonate salt and/or carboxybenzene diazonium salt.

According to one embodiment, the conditions enabling the formation of radical entities in the process of the invention may be obtained by using an activator, for example by varying the temperature and/or by adding a chemical activator and/or by using a photochemical and/or radiochemical environment.

According to a first embodiment, the conditions enabling the formation of radical entities may be obtained by adding in the solution used in the process of the present invention a reducing agent as chemical activator. The reducing agent may be for example ascorbic acid, hypophosphoric acid, iron fillings.

In a second embodiment, conditions enabling the formation of radical entities may be obtained by using a temperature ranging from 20°C to 90°C, preferably from 30°C to 60°C, more preferably about 40°C.

According to an embodiment, the amount of chemical activator in the solution used in the process of the present invention is ranging from 0.001 M to 0.5 M, preferably from 0.002 M to 0.1 M, more preferably from 0.002 M to 0.01 M. This amount has to be chosen according to the conditions used. Preferably, this amount represents from 0.1 to 20 times of the diazonium salt concentration, as a function of the nature of the chemical activator.

According to one embodiment, the solvent of the reaction is a protic solvent. In an embodiment, the protic solvent is chosen from the group comprising water, deionized water, distilled water, acidified or not, acetic acids, hydroxylated solvents such as methanol and ethanol, low-molecular-weight liquid glycols such as ethyleneglycol and mixtures thereof. In a preferred embodiment, the protic solvent is water, deionised water or distilled water, acidified or not.

According to another embodiment, the solvent of the reaction is an aprotic solvent, preferably acetonitrile, dimethylformamide, dimethylsulfoxide or a mixture thereof.

Alternatively, the solvent of the reaction is a mixture of a protic solvent or a mixture of protic solvents together with an aprotic solvent or a mixture of aprotic solvents.

According to one embodiment, the pH of the solution used in the process of the present invention is less than 7, preferably less than or equal to 3.

According to an embodiment, a surfactant may be added in the solution used in the process of the present invention. According to the present invention, a surfactant is a molecule comprising a lipophilic part (apolar) and a hydrophilic part (polar). Without willing to be linked by any theory, it is Applicant opinion that the presence of a surfactant promotes radicals formation by isolating them in micelles and therefore promotes copolymerization-like reaction. Among surfactants that may be used according to the present invention, it is possible to mention:
i) anionic surfactants, in which the hydrophilic part is negatively charged, such as for example tetraethylammonium para-toluene sulfonate, sodium dodecylsulfate, sodium palmitate, sodium stearate, sodium myristate, di(2-ethylhexyl) sodium sulfosuccinate, methylbenzene sulfonate and ethylbenzene sulfonate;
ii) cationic surfactants, in which the hydrophilic part is positively charged, such as for example ammonium tetradecyl trimethyl bromide (TTAB), alkyl-pyridinium halides having a C1-C18 aliphatic chain and the alkylammonium halides;
iii) zwitterionic surfactants which are neutral compounds having formal electrical charges with similar value and opposite sign, such as for example N,N-dimethyldocecyl ammonium sodium butanoate, dimethyldodecyl ammonium sodium propanoate, and the amino acids;
iv) amphoteric surfactants, which are compounds that simultaneously behave like an acid or like a base depending on the medium in which they are placed; these compounds may have a zwitterionic nature, amino acids are specific example of this family;
v) neutral surfactants, also called non-ionic surfactants, wherein the surfactant properties, in particular hydrophobicity, are provided by uncharged functional groups, such as for example polyethers like the polyethoxylated surfactants such as e.g. polyethylene glycol lauryl ether (POE23 or Brij(R) 35), the polyols (surfactants derived from sugars), in particular the glucose alkylates such as e.g., glucose hexanate.

The process of the present invention is carried out under gentle and non-destructive conditions, preferably under normal conditions of temperature and pressure.

According to one embodiment, the material to be grafted is immersed the solution used in the process of the invention. According to another embodiment, the solution is sprayed onto the surface of the material.

According to one embodiment, the reaction is performed during a period of time ranging from 5 min to 90 min, preferably from 10 min to 30 min.

According to an embodiment, the reaction time may be adjusted. This adjustment of the time of exposure of the surface of the material to the solution makes it possible to control the thickness of the film that is obtained.

According to one embodiment, the efficiency of the grafting may be determined by any suitable means of analysis, especially by X photoelectron spectroscopy (XPS) measurements or measure of contact angles. According to one embodiment, XPS analysis may be performed using a Kratos Axis Ultra apparatus. According to one embodiment, contact angle measure may be performed using an Apollo Instruments apparatus.

According to an embodiment, the process of the present invention comprises a preliminary step of pre-treating the surface of the material to be grafted. In this embodiment, the pre-treatment comprises cleaning the surface to be grafted, for example by ultrasound treatment in water and/or in an organic solvent such as cyclohexane, ethanol. Prior to grafting, the pre-treated surface may be further rinsed with water, preferably deionized water.

According to another embodiment, the surface of the material may be pre-treated by an acidic treatment, a basic treatment or an oxido-reductive treatment.

According to an embodiment, the process of the present invention comprises a further step of post-treatment. This further step comprises treating the grafted material in water at a temperature ranging from 60 to 100°C, preferably about 100°C for a period of time ranging from 1 to 10, preferably about 5 min, optionally followed by rinsing in a solvent such as ethanol. Without willing to be linked by any theory, it is Applicant's opinion that this post-treatment allows to eliminate the majority of non-grafted compounds. This step avoids therefore the release of non-grafted compounds once the grafted implantable material is implanted.

### Use of grafted implantable materials

The present invention also relates to the use of the grafted implantable material of the invention to manufacture an antiproliferative and/or antibacterial implantable medical device, preferably an antiproliferative and/or antibacterial IOL.

Implantable medical devices that may be grafted by the process of the present invention are for example implants, intraocular lenses (IOLs), stents, catheters, implants for abdominal surgery, vascular prostheses, artificial limbs, preferably IOLs.

According to one embodiment, implantable medical devices that may be grafted by the process of the present invention are non-metallic.

According to one embodiment, the grafted implantable material of the invention is used to manufacture an antiproliferative and/or antibacterial IOL. In one aspect of this embodiment, the implantable material to be grafted by the process of the present invention is hydrophilic or hydrophobic. In another aspect of this embodiment, the implantable material to be grafted by the process of the present invention is an IOL, preferably a commercially available IOL.

The present invention also relates to a kit comprising a grafted implantable material according to the present invention and an inserting and/or implantation device.

According to one embodiment, the kit of the invention comprises an intraocular lens (IOL) grafted according to the invention and an IOL-inserting device.

The present invention also relates to an intraocular lens having at least one external surface grafted with a polymeric film comprising carboxylate and sulfonate functions wherein the film is produced by copolymerization-like reaction of a source of carboxylate functions and a source of sulfonate functions.

### BRIEF DESCRIPTION OF THE DRAWINGS

**FIG. 1** is an infrared spectrum of a gold substrate grafted according to the present invention using 4-sulfoxybenzene diazonium salt adhesion primer as source of sulfonate functions.
**FIG. 2** is an infrared spectrum of a gold substrate grafted according to the present invention using 4-sulfoxybenzene diazonium salt adhesion primer as source of sulfonate functions and acrylic acid polymerizable monomer as source of carboxylate functions.
**FIG. 3** is an infrared spectrum of a gold substrate grafted according to the present invention using 4-sulfoxybenzene diazonium salt adhesion primer and sodium styrene sulfonate polymerizable monomer as source of sulfonate functions and acrylic acid polymerizable monomer as source of carboxylate functions.
**FIG. 4** is an infrared spectrum of a gold substrate grafted according to the present invention using 4-carboxybenzene diazonium salt adhesion primer as source of carboxylate functions.
**FIG. 5** is an infrared spectrum of a gold substrate grafted according to the present invention using 4-carboxybenzene diazonium salt adhesion primer as source of carboxylate functions and sodium styrene sulfonate polymerizable monomer as source of sulfonate functions.
**FIG. 6** is an infrared spectrum of a gold substrate grafted according to the present invention using 4-carboxybenzene diazonium salt adhesion primer as source of carboxylate functions and sodium styrene sulfonate polymerizable monomer as source of sulfonate functions.
**FIG. 7** is an infrared spectrum of a gold substrate grafted according to the present invention using 4-carboxybenzene diazonium salt adhesion primer and acrylic acid polymerizable monomer as source of carboxylate functions and sodium styrene sulfonate polymerizable monomer as source of sulfonate functions.
**FIG. 8** is an infrared spectrum of a gold substrate grafted according to the present invention using 4-carboxybenzene diazonium salt adhesion primer as source of carboxylate functions and 4-sulfoxybenzene diazonium salt adhesion primer as source of sulfonate functions.
**FIG. 9** is an infrared spectrum of a gold substrate grafted according to the present invention using 4-carboxybenzene diazonium salt adhesion primer as source of carboxylate functions and sodium styrene sulfonate polymerizable monomer as source of sulfonate functions, in the absence of reducing agent.
**FIG. 10** is an infrared spectrum of the 4-carboxybenzene diazonium tetrafluoroborate.
**FIG. 11** is an XPS spectra of a PHEMA cylinder grafted according to the present invention using 4-carboxybenzene diazonium salt adhesion primer as source of carboxylate functions and sodium styrene sulfonate polymerizable monomer as source of sulfonate functions.
**FIG. 12** is an XPS spectra of an hydrophobic intraocular implant grafted according to the present invention using 4-carboxybenzene diazonium salt adhesion primer as source of carboxylate functions and sodium styrene sulfonate polymerizable monomer as source of sulfonate functions.
**FIG. 13** is a graph representing the number of LEC cells present at the surface of materials grafted according to the present invention (Statistics: Control vs Test; ** p<0.001; *** p<0.0001).
**FIG. 14** is a scheme representing the principles of radical copolymerization and of Graftfast® copolymerization-like reaction.

### EXAMPLES

The present invention is further illustrated by the following examples.

### Materials

The experiments were conducted in deionized water (DI water) at room temperature. All standard chemicals were purchased from Sigma Aldrich.

The following examples were performed in glass cell and otherwise stated they were conducted in normal conditions of temperature and pressure in ambient air.

Infrared spectra were obtained on a Bruker VERTEX 70 spectrometer equipped with ATR Pike-Miracle device. The detector was a MCT working at liquid nitrogen temperature. The spectra were obtained after 256 scans at 2 cm⁻¹ resolution and contributions from H₂O and CO₂ (gas) were subtracted.

XPS analyses were performed with a Kratos Axis Ultra DLD using a high-resolution monochromatic Al-Kα line X-ray source at 1486.6 eV. Fixed analyzer pass energy of 20 eV was used for core level scans. The photoelectron take-off angle was always normal to the surface, which provided an integrated sampling depth of approximately 15 nm. A survey spectrum and core-level spectra of C1s (280 - 290 eV), O1s (526 - 538 eV) and N1s (396 - 410 eV) regions were systematically recorded. The energy scale of the instrument was calibrated by setting Au 4f_{7/2} = 84.00 eV, Ag3d_{5/2} = 368.70 eV, CuL₃M_{4,5}M_{4,5} = 567.90 eV and Cu 2p_{3/2} = 932.65 eV. When charging phenomena occurred, the charge was counterbalanced by adjusting the Au 4f_{7/2} level of the pristine gold substrate (reference sample always analyzed if the charge neutralizer was employed) at 84.00 eV and by applying this shift to all the samples studied in the same batch. Spectra were treated with Avantage software.

Contact angle measurements were made on a system from Apollo Instruments by delivering a 2 µL drop of ultra-pure water (H₂O MQ 18 MΩ) at a 1 µL/s speed from a microsyringe onto the sample mounted on an illuminated horizontal stage. The image of the static water droplet was captured by a video camera and the SCA20 software. Six measurements were taken for each sample and the average water contact angle was calculated.

To study the composition of spontaneously grafted films, substrates were immersed for 1 h in aqueous (H₂O DI pH = 5.5) solutions containing the reactants. The substrates were analyzed by IR-ATR (Attenuated Total Reflection), XPS (X-ray photoemission spectroscopy) and contact angle after a rinsing procedure consisting in a simple wash with water and ethanol expected to remove all the non-grafted species from the surface.

### 1. Determination of the reactants

Tests were performed on gold substrates in order to study the reactivity of different adhesion primers and polymerizable monomers and to determine their suitability to graft carboxylate and sulfonate functions on a substrate with the Graftfast^{®} technology.

### 1.1. Use of 4-sulfoxybenzene diazonium as adhesion primer

### 1.1.1. Reactivity of 4-sulfoxybenzene diazonium salt

The reactivity of 4-sulfoxybenzene diazonium salt as adhesion primer was tested on gold substrates.

Sulfanilic acid (433 mg) is dissolved in 0.5 M HCl (25mL). A solution of NaNO₂ (172.5 mg) in deionized water (25 mL) is added dropwise under mechanical agitation in the sulfanilic solution. Gold substrates are placed in the solution, under agitation and ascorbic acid (44.03 mg) is added in the mixture. The reaction is let for 30 minutes. A light yellowing of the solution occurs as well as a gaseous release.

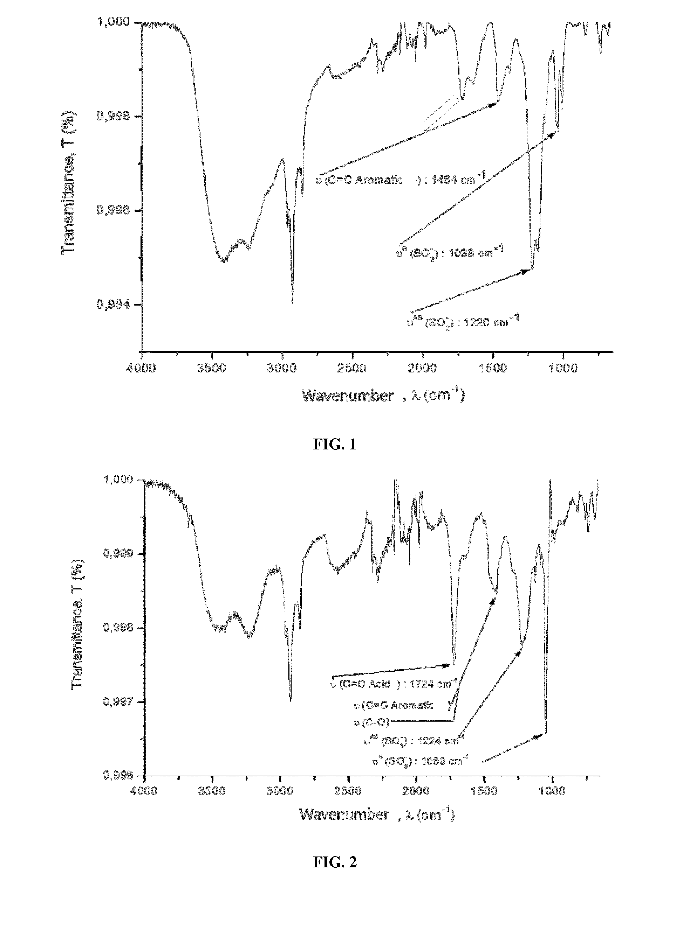

The gold substrates are rinsed (water, ethanol, and DMF under ultrasonics for 2 min) and dried under nitrogen flux. Grafted samples are analyzed by infrared spectroscopy (figure 1). The characteristic absorption bands are the symmetric vibration band of the-SO₃⁻ group at 1038 and 1220 cm⁻¹. The intensities (in percentages of transmittance) of these two bands for three similarly treated samples are the following:

| | SO₃⁻ (AS) | SO₃⁻ (S) |
|---|---|---|
| Sample 1 | 0.38 | Not readable |
| Sample 2 | 0.26 | 0.12 |
| Sample 3 | 0.47 | 0.17 |

The characteristic sulfonate absorption band revealed by infrared spectroscopy attests of the grafting of the expected sulfonate functions on the surface of the gold substrates when using 4-sulfoxybenzene diazonium salt as adhesion primer.

### 1.1.2. Use of acrylic acid as polymerizable vinylic monomer

The grafting of carboxylate and sulfonate functions using a 4-sulfoxybenzene diazonium salt as adhesion primer and acrylic acid as polymerizable monomer was tested on gold substrates.

Acrylic acid (0.75 mol/L) and sulfanilic acid (0.05 mol/L) are mixed in deionized water, acidified with HCl (pH = 2). NaNO₂ (0.05 mol/L) is added dropwise under mechanical agitation. Gold substrates are placed in the solution, under agitation and ascorbic acid (0.005 mol/L) is added in the mixture. The reaction is let for 30 minutes. A light yellowing of the solution occurs as well as a gaseous release.

The gold substrates are rinsed (water, ethanol, and DMF under ultrasonics for 2 min) and dried under nitrogen flux. Grafted samples are analyzed by infrared spectroscopy (figure 2). The two characteristic absorption bands are the carbonyl band C=O at 1724 cm⁻¹ and the symmetric vibration band of the -SO₃⁻ group. The intensities (in percentages of transmittance) of these two bands for three similarly treated samples are the following:

| | C=O | SO₃⁻ |
|---|---|---|
| Sample 1 | 0.06 | 0.34 |
| Sample 2 | 0.1 | 0.33 |
| Sample 3 | 0.19 | 0.27 |

The characteristic carboxylate and sulfonate absorption bands, revealed by infrared spectroscopy, attest of the grafting of the expected carboxylate and sulfonate functions on the surface of the gold substrates.

### 1.1.3. Use of acrylic acid and sodium styrene sulfonate as polymerizable vinylic monomers

The grafting of carboxylate and sulfonate functions using a 4-sulfoxybenzene diazonium salt as adhesion primer and a mixture of acrylic acid and sodium styrene sulfonate as polymerizable monomers was tested on gold substrates.

Sulfanilic acid (476.3 mg) is dissolved in 0.5M HCl (25 mL). A solution of NaNO₂ (189.8 mg) in deionized water (25 mL) is added dropwise under mechanical agitation to the sulfanilic solution. Acrylic acid (1.43 mL) and sodium styrene sulfonate (4.253 g) are added in the solution and the reaction volume is adjusted to 55 mL with deionized water. Gold substrates are placed in the solution and ascorbic acid (48.5 mg) is added in the mixture. The reaction is let for 30 minutes. A light yellowing of the solution occurs as well as a gaseous release.

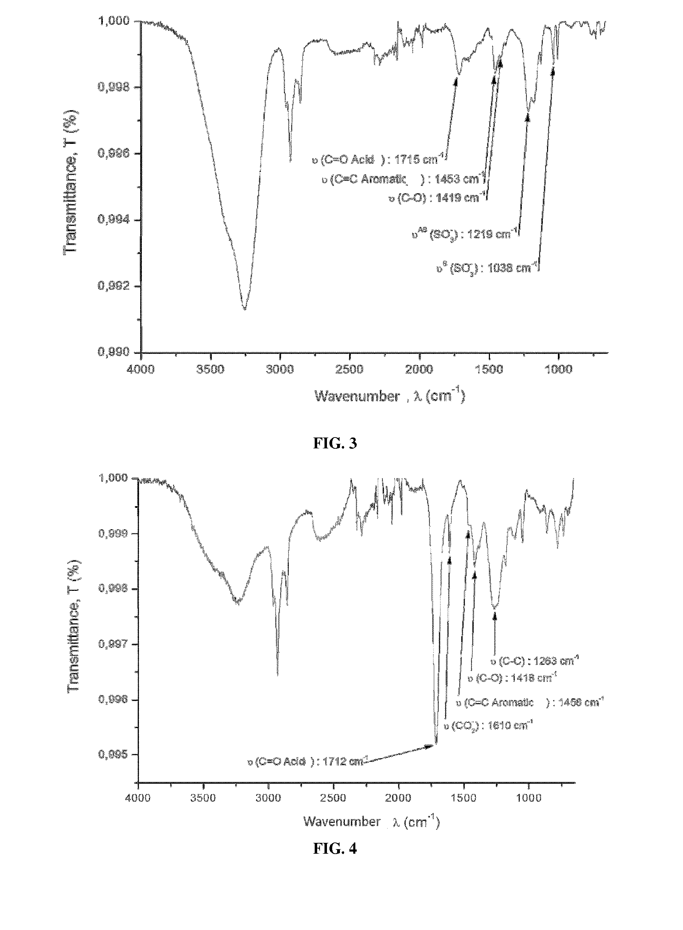

The gold substrates are rinsed (water, ethanol, and DMF under ultrasonics for 2 min) and dried under nitrogen flux. Grafted samples are analyzed by infrared spectroscopy (figure 3). The two characteristic absorption bands are the carbonyl band C=O at 1715 cm⁻¹ and the symmetric vibration band of the -SO₃⁻ group. The intensities (in percentages of transmittance) of these two bands for three similarly treated samples are the following:

| | C=O | SO₃⁻ |
|---|---|---|
| Sample 1 | 0.15 | 0.08 |
| Sample 2 | 0.18 | 0.07 |
| Sample 3 | 0.14 | 0.12 |

The characteristic carboxylate and sulfonate absorption bands, revealed by infrared spectroscopy, attest of the grafting of the expected carboxylate and sulfonate functions on the surface of the gold substrates.

### 1.2. Use of 4-carboxybenzene diazonium as adhesion primer

### 1.2.1. Reactivity of 4-carboxybenzene diazonium salt

The reactivity of 4-carboxybenzene diazonium salt as adhesion primer was tested on gold substrates.

4-Aminobenzoic acid (342.9 mg) is dissolved in 0.5 M HCl (25mL). A solution of NaNO₂ (172.5 mg) in deionized water (25 mL) is added dropwise under mechanical agitation in the sulfanilic solution. Gold substrates are placed in the solution, under agitation and ascorbic acid (44.03 mg) is added in the mixture. The reaction is let for 30 minutes. A light yellowing of the solution occurs as well as a gaseous release.

The gold substrates are rinsed (water, ethanol, and DMF under ultrasonics for 2 min) and dried under nitrogen flux. Grafted samples are analyzed by infrared spectroscopy (figure 4). The characteristic absorption bands is the carbonyl band C=O at 1712 cm⁻¹ The intensities (in percentages of transmittance) of this band for two similarly treated samples are the following:

| | C=O |
|---|---|
| Sample 1 | 0.49 |
| Sample 2 | 0.43 |

The characteristic carboxylate absorption band revealed by infrared spectroscopy attests of the grafting of the expected carboxylate function on the surface of the gold substrates when using 4-carbobenzene diazonium salt as adhesion primer.

### 1.2.2. Use of sodium styrene sulfonate as polymerizable vinylic monomer

### 1.2.2.1. Concentration 0.75 mol/L

Sodium styrene sulfonate (0.75 mol/L) and 4-aminobenzoic acid (0.05 mol/L) are mixed in deionized water, acidified with HCl (pH = 2). NaNO₂ (0.05 mol/L) is added dropwise under mechanical agitation. Gold substrates are placed in the solution, under agitation and ascorbic acid (0.005 mol/L) is added in the mixture. The reaction is let for 30 minutes. A light yellowing of the solution occurs as well as a gaseous release.

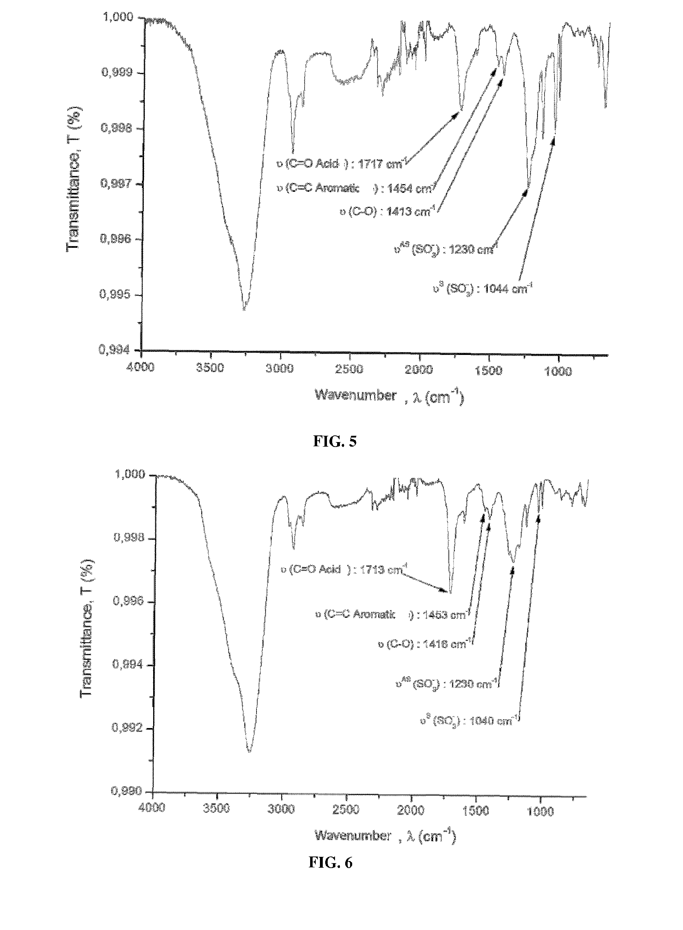

The gold substrates are rinsed (water, ethanol, and DMF under ultrasonics for 2 min) and dried under nitrogen flux. Grafted samples are analyzed by infrared spectroscopy (figure 5). The two characteristic absorption bands are the carbonyl band C=O at 1717 cm⁻¹ and the symmetric vibration band of the -SO₃⁻ group. The intensities (in percentages of transmittance) of these two bands for three similarly treated samples are the following:

| | C=O | SO₃⁻ |
|---|---|---|
| Sample 1 | 0.12 | 0.18 |
| Sample 2 | 0.14 | 0.28 |
| Sample 3 | 0.21 | 0.18 |

The characteristic carboxylate and sulfonate absorption bands, revealed by infrared spectroscopy, attest of the grafting of the expected carboxylate and sulfonate functions on the surface of the gold substrates.

### 1.2.2.2. Concentration 0.5 mol/L

The experimental conditions are the same as in paragraph 1.2.2.1 above, aside the concentration of sodium styrene sulfonate added in the solution that is of 0.5 mol/L instead of 0.75 mol/L.

Grafted samples are analyzed by infrared spectroscopy (figure 6). The two characteristic absorption bands are the carbonyl band C=O at 1713 cm⁻¹ and the symmetric vibration band of the -SO₃⁻ group. The intensities (in percentages of transmittance) of these two bands for four similarly treated samples are the following:

| | C=O | SO₃⁻ |
|---|---|---|
| Sample 1 | 0.35 | 0.22 |
| Sample 2 | 0.35 | 0.18 |
| Sample 3 | 0.27 | 0.23 |
| Sample 4 | 0.32 | 0.17 |

The characteristic carboxylate and sulfonate absorption bands, revealed by infrared spectroscopy, attest of the grafting of the expected carboxylate and sulfonate functions on the surface of the gold substrates.

### 1.2.2.3. Effect of the concentration

The means of the intensities (in percentages of transmittance) of the two bands for the two tests above, respectively with a concentration of 0.75 and 0.5 mol/L of sodium styrene sulfonate, are summarized below:

| | C=O | SO₃⁻ | ratio [COO]/[SO₃] |
|---|---|---|---|
| mean for 0.75 mol/L | 0.16 | 0.21 | 0.76 |
| mean for 0.5 mol/L | 0.32 | 0.20 | 1.60 |

The ratio between the two chemical functions grafted on the surface may therefore be modulated by varying the concentration in sodium styrene sulfonate.

### 1.2.3. Use of acrylic acid and sodium styrene sulfonate as polymerizable vinylic monomers

The grafting of carboxylate and sulfonate functions using a 4-caboxybenzene diazonium salt as adhesion primer and a mixture of acrylic acid and sodium styrene sulfonate as polymerizable monomers was tested on gold substrates.

4-aminobenzoic acid (377.1 mg) is dissolved in 0.5M HCl (25 mL). A solution of NaNO₂ (189.8 mg) in deionized water (25 mL) is added dropwise under mechanical agitation to the aminobenzoic acid solution. Acrylic acid (1.43 mL) and sodium styrene sulfonate (4.253 g) are added in the solution and the reaction volume is adjusted to 55 mL with deionized water. Gold substrates are placed in the solution and ascorbic acid (48.5 mg) is added in the mixture. The reaction is let for 30 minutes. A light yellowing of the solution occurs as well as a gaseous release.

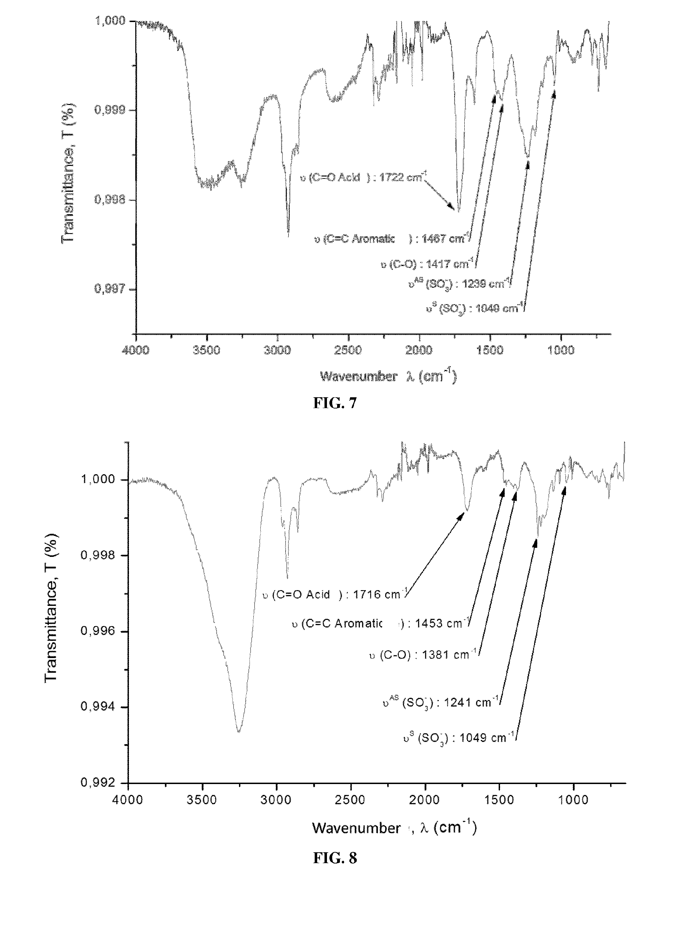

The gold substrates are rinsed (water, ethanol, and DMF under ultrasonics for 2 min) and dried under nitrogen flux. Grafted samples are analyzed by infrared spectroscopy (figure 7). The two characteristic absorption bands are the carbonyl band C=O at 1722 cm⁻¹ and the symmetric vibration band of the -SO₃⁻ group. The intensities (in percentages of transmittance) of these two bands for three similarly treated samples are the following:

| | C=O | SO₃⁻ |
|---|---|---|
| Sample 1 | 0.19 | 0.06 |
| Sample 2 | 0.17 | 0.06 |
| Sample 3 | 0.09 | not readable |

The characteristic carboxylate and sulfonate absorption bands, revealed by infrared spectroscopy, attest of the grafting of the expected carboxylate and sulfonate functions on the surface of the gold substrates.

### 1.3. Use of 4-carboxybenzene diazonium and 4-sulfoxybenzene diazonium as adhesion primers

The reactivity of a mixture of 4-sulfoxybenzene diazonium salt and 4-carboxybenzene diazonium salt as adhesion primers was tested on gold substrate.

Sulfanilic acid (909.3 mg) is dissolved in 0.5M HCl (25 mL). 4-Aminobenzoic acid (720 mg) is dissolved in 0.5M HL (25 mL). The solution of 4-aminobenzoic acid is mixed with the solution of sulfanilic acid. A solution of NaNO₂ (1,449 g) in deionized water (50 mL) is added dropwise under agitation in the mixture. Gold substrates are placed in the solution, under agitation and ascorbic acid (184.9 mg) in water (5 mL) is added in the mixture. The reaction is let for 30 minutes and a gaseous release occurs.

The gold substrates are rinsed (water, ethanol, and DMF under ultrasonics for 2 min) and dried under nitrogen flux. Grafted samples are analyzed by infrared spectroscopy (figure 8). The intensities (in percentages of transmittance) of characteristic bands for three similarly treated samples are the following:

| | C=O | SO₃⁻ (AS) | SO₃⁻ (S) |
|---|---|---|---|
| Sample 1 | 0.15 | 0.2 | 0.04 |
| Sample 2 | 0.15 | 0.11 | not readable |
| Sample 3 | 0.14 | 0.08 | not readable |

The characteristic sulfonate and carboxylate absorption bands, revealed by infrared spectroscopy, attest of the grafting of the expected sulfonate and carboxylate functions on the surface of the gold substrates.

### 1.4. Reaction in absence of reducing agent

The grafting was tested in absence of reducing agent but using temperature as activator.

Sodium styrene sulfonate (0.5 mol/L) and 4-aminobenzoic acid (0.05 mol/L) are mixed in deionized water, acidified with HCl (pH = 2). NaNO₂ (0.05 mol/L) is added dropwise under mechanical agitation. Gold substrates are placed in the solution, under agitation and at 55°C. The reaction is let for 1 hour at 55°C under agitation.

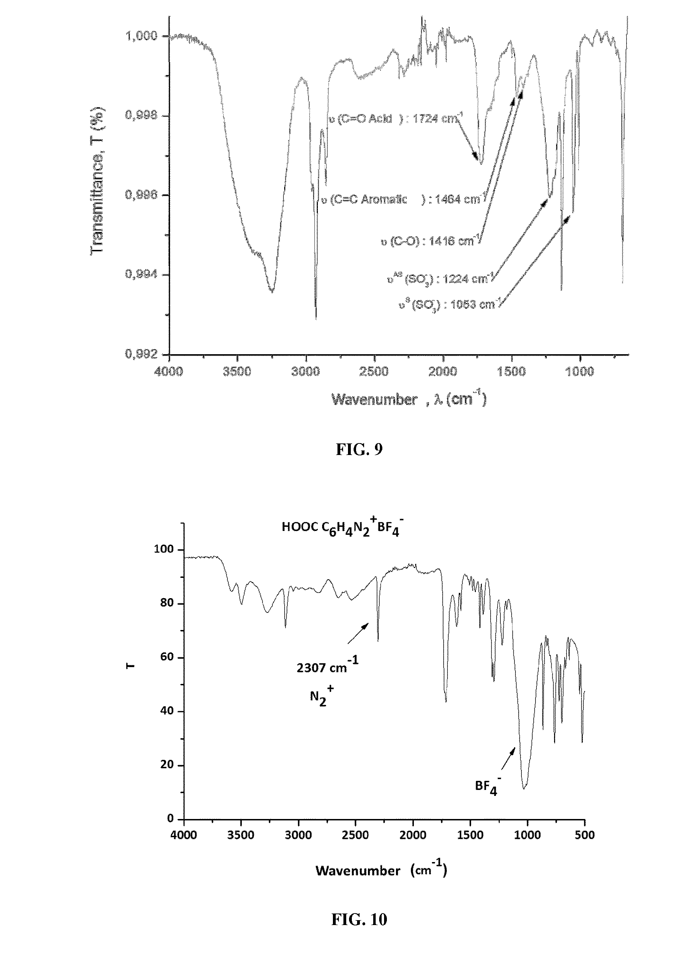

The gold substrates are rinsed (water, ethanol, and DMF under ultrasonics for 2 min) and dried under nitrogen flux. Grafted samples are analyzed by infrared spectroscopy (figure 9). The intensities (in percentages of transmittance) of characteristic bands for three similarly treated samples are the following:

| | C=O | SO₃⁻ (AS) | SO₃⁻ (S) |
|---|---|---|---|
| Sample 1 | 0.32 | 0.36 | 0.35 |
| Sample 2 | 0.27 | 0.59 | 1.08 |
| Sample 3 | 0.2 | 0.3 | 0.28 |

The characteristic carboxylate and sulfonate absorption bands, revealed by infrared spectroscopy, attest of the grafting of the expected sulfonate and carboxylate functions on the surface of the gold substrates.

### 2. IOL grafting

### 2.1. Synthesis of 4-carboxybenzene diazonium tetrafluoroborate

The route of synthesis is the following:

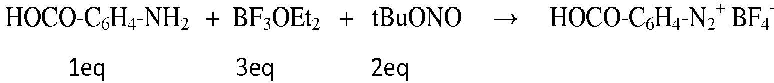

In a 50 mL flask is introduced aminobenzoic acid (3.04g, 0.022 mol) dissolved in THF (10 mL). The solution is placed on an acetonitrile bath cooled with liquid nitrogen at -30°C. BF₃OEt₂ (8.1 mL, 0.065 mol) is added through a syringe and the mixture is stirred 20 minutes at -40°C. BuONO (5.1 mL, 0.044 mol) is added dropwise. The mixture is stirred 10 minutes at -40°C after the end of the addition and the colds bath is then let to reach room temperature. The formed salt is precipitated in cold ether (250 mL) and filtrated to give a white solid (3.37 g, yield = 66%). The infrared spectra of the product is given in figure 10.

### 2.2. Hydrophilic plots grafting

Cylinders of polyhydroxyethyl methacrylate (PHEMA) having a diameter of 13 mm and a high of 3 mm were used for grafting tests. These cylinders are precursors of hydrophilic intraocular implants.

### 2.2.1. Diazonium salt one-pot synthesis

The grafting of 12 PHEMA cylinders was tested in presence of sodium styrene sulfonate and 4-aminobenzoic acid with the one pot synthesis of the corresponding diazonium salt.

### Pre-treatment

The PHEMA cylinders were placed in a beaker, covered by cyclohexane and treated by ultrasonic for 1 minute. The cylinders were then dried under nitrogen flux.

Sodium styrene sulfonate (0.5 mol/L) and 4-aminobenzoic acid (0.05 mol/L) are mixed in deionized water, acidified with HCl (pH = 2). NaNO₂ (0.05 mol/L) is added dropwise under mechanical agitation. PHEMA cylinders are placed in the solution and ascorbic acid (0.005 mol/L) is added in the mixture. The reaction is let for 30 minutes. The PHEMA cylinders are then rinsed with acetone and water and dried under a nitrogen flux.

### 2.2.2. Use of the isolated diazonium salt

The grafting of two series of 25 PHEMA cylinders (batch 1 and batch 2) was tested in presence of sodium styrene sulfonate and 4-carboxybenzene diazonium tetrafluoroborate.

### Pre-treatment

The PHEMA cylinders were placed in a beaker, covered by cyclohexane and treated by ultrasonic for 1 minute. The cylinders were then dried under nitrogen flux.

### Grafting

4-carboxybenzene diazonium tetrafluoroborate (3.5 g, 0.0148 mol)) was dissolved in 290 mL of deionized water. Sodium styrene sulfonate (30.86 g, 0.15 mol) were added in the solution. The 25 cylinders of one batch were immersed in the solution. Ascorbic acid (0.265 g, 0.0015 mol) dissolved in 10 mL of deionized water was then added in the solution. The reacting mixture switched from yellow to dark brown. The reaction is performed for 30 minutes. The cylinders were then rinsed with deionized water and placed for 2 hours in a beaker full of deionized water. The cylinders were then dried under nitrogen flux and put in a desiccator for one night.

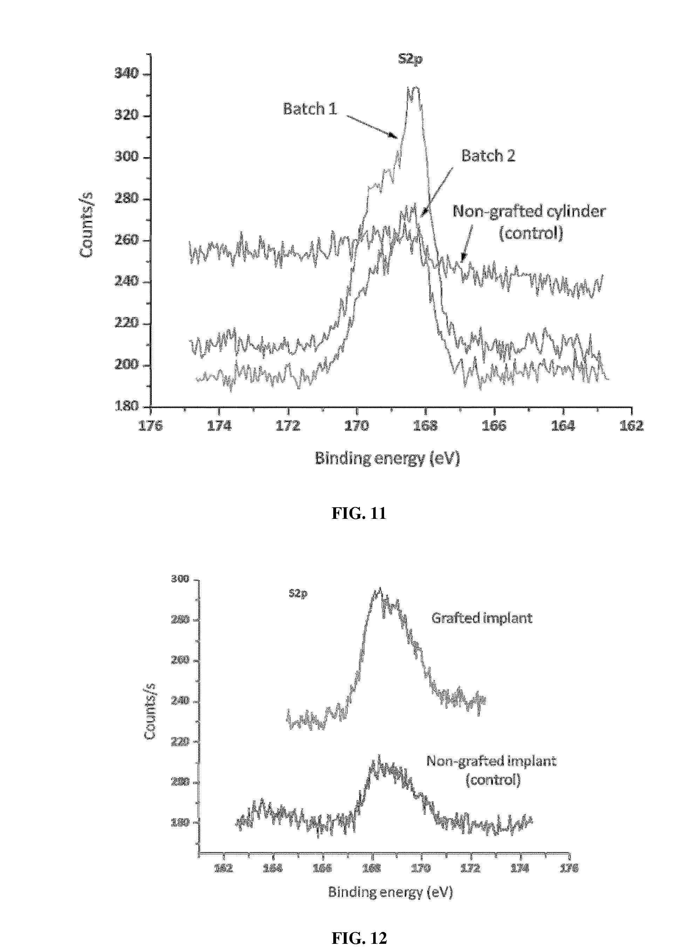

The surface of the grafted cylinders was analyzed by X-photoelectrons spectroscopy (XPS) and compared with the one of a non-grafted cylinder. Among the elements carbon, oxygen, nitrogen and sulfur, it is the sulfur that reveal the presence of polystyrene sulfonate on the grafted surface. The XPS spectra of the levels of sulfur heart are represented on figure 11. The carboxylic groups cannot be differentiated from the esters groups present in the PHEMA constitutive of the cylinder.

On figure 11, it is clear that the sulfur content of non-grafted cylinders is negligible whereas it is well marked on the two grafted samples. This analysis assesses the presence of -SO₃⁻ functions grafted on the surface of the PHEMA.

### 2.3. Hydrophobic implants grafting

The grafting of a hydrophobic intraocular implant was tested in presence of sodium styrene sulfonate and 4-aminobenzoic acid with the one pot synthesis of the corresponding diazonium salt.

The exact nature of the tested commercial implant is unknown (mixture of methacrylates and additives) but has a hydrophobic surface.

A series of 9 implants was grafted.

### Pre-treatment

As the surface of the implants is hydrophobic, an acidic treatment was performed in order to render the surface more hydrophilic. The treatment consists in gently rubbing the surface of the implant with a lint-free cloth moisture with 100% acetic acid.

### Grafting

The grafting of the 9 implants is performed according to the method described at paragraph 1.2.2.2 with the one-pot synthesis of the diazonium salt.

The reaction is performed for 30 minutes. The implants were then rinsed with deionized water and placed for 1 hour in a beaker full of deionized water. The cylinders were then dried under nitrogen flux.

The surface of the grafted implants was analyzed by X-photoelectrons spectroscopy (XPS) and compared with the one of a non-grafted implant. As for hydrophilic cylinders above, only the level of the sulfur heart (S2p level) is studied, as represented on figure 12.

As evidenced on figure 12, the non-grafted implant already comprises sulfur, with about 30 counts/s for the S2p level. The grafting allows doubling the sulfur amount, with about 60 counts/s.

### 3. Cellular proliferation assessment

### 3.1. Method

Study of the growth of human lens cells (LEC) onto grafted material obtained according to the methods described at paragraphs 2.2 and 2.3 above.

Human eye lens epithelial cells (LEC, CRL-11421,ATCC, USA) are seeded onto grafted implants in 24-well microplates at a rate of 50 000 cells per well in RPMI medium (VWR, France). After 1, 3 and 7 days of culture, LEC cells are counted using the MTT colorimetric assay. Control corresponds to LEC culture onto non-grafted material.

### 3.2. Results

A general observation is that human lens cells do not proliferate onto grafted materials and that grafted materials of the invention have cytostatic but no cytotoxic activities. This is essential as if the material was cytotoxic, dead cells would form a layer on the surface and opacification would not be avoided as expected.

### 3.2.1. Grafted hydrophilic plots

As evidence on figure 13, there is no significant difference for cell proliferation between batches 1 and 2.

### 3.2.2. Grafted hydrophobic implants

As evidence on figure 13, a weak proliferation was observed on grafted hydrophobic implants.

## Claims

1. Implantable material having at least one external surface grafted with a polymeric film comprising carboxylate and sulfonate functions wherein the film is produced by copolymerization-like reaction of a source of carboxylate functions and a source of sulfonate functions.

2. Implantable material according to claim 1, wherein one source of carboxylate functions is an aromatic adhesion primer comprising at least one carboxylic acid function or at least one carboxylate salt function.

3. Implantable material according to claim 1 or claim 2, wherein one source of sulfonate functions is an aromatic polymerizable monomer, preferably an aromatic polymerizable vinylic monomer, comprising at least one sulfonic acid function or at least one sulfonate salt function.

4. Implantable material according to anyone of claims 1 to 3, wherein one source of carboxylate functions is a diazonium salt used as adhesion primer, preferably a carboxybenzene diazonium salt, preferably a 4-carboxybenzene diazonium salt, more preferably sodium 4-carboxybenzene diazonium or 4-carboxybenzene diazonium tetrafluoroborate.

5. Implantable material according to anyone of claims 1 to 4, wherein one source of sulfonate functions is a styrene derivative use as polymerizable vinylic monomer, preferably a styrene sulfonate salt, preferably sodium 4-styrene sulfonate.

6. Implantable material according to anyone of claims 1 to 5, wherein the film is produced by copolymerization-like reaction of a 4-carboxybenzene diazonium salt and a styrene sulfonate salt, preferably by copolymerization-like reaction of sodium 4-carboxybenzene diazonium and sodium 4-styrene sulfonate or of 4-carboxybenzene diazonium tetrafluoroborate and sodium 4-styrene sulfonate.

7. Implantable material according to anyone of claims 1 to 6, wherein the thickness of the polymeric film is ranging from 1 nm to 50 nm, preferably from 2 nm to 20 nm.

8. Implantable material according to anyone of claims 1 to 7, wherein the ratio between the number of carboxylate functions and sulfonate functions [COO⁻]/[SO₃⁻] is less than 10, preferably less than 5, more preferably ranging from 0.5 to 2, more preferably ranging from 0.7 to 1.3, more preferably ranging from 0.9 to 1.1.

9. A process for grafting of a polymeric film onto at least one external surface of an implantable material, comprising a step of contacting said external surface with a solution comprising a source of carboxylate functions, preferably a carboxybenzene diazonium salt, and a source of sulfonate functions, preferably a styrene sulfonate salt, in a solvent, under conditions enabling the formation of radical entities.

10. The process according to claim 9, wherein the solvent is water, deionized water, distilled water, acidified or not, acetic acids, hydroxylated solvents such as ethanol, low-molecular-weight liquid glycols such as ethyleneglycol and mixtures thereof.

11. The process according to claim 9 or claim 10, wherein the conditions enabling the formation of radical entities comprise the use of a reducing agent, preferably ascorbic acid.

12. The process according to anyone of claims 9 to 11 wherein the carboxybenzene diazonium salt is obtained in situ from aminobenzoic acid by a diazotation reaction, preferably in presence of sodium nitrite.

13. Use of an implantable material according to anyone of claims 1 to 8 for the manufacture of an antiproliferative and/or antibacterial implantable medical device, preferably an antiproliferative and/or antibacterial implant, more preferably an antiproliferative and/or antibacterial intraocular lens.

14. Kit comprising an implantable material according to anyone of claims 1 to 8 and an inserting and/or implantation device, preferably wherein the implantable material is an intraocular lens and the implantation device is an IOL-inserting system.
